# EUROPEAN PATENT APPLICATION

(11) **EP 4 289 353 A1**
(43) Date of publication of application: **13.12.2023**
(21) Application number: 22853572.0
(22) Date of filing: 08.08.2022
(51) Int. Cl.: A61B 5/145, A61B 5/00, A61B 5/107, G16H 50/20, G16H 50/50

(54) **ARTIFICIAL-INTELLIGENCE-BASED BLOOD GLUCOSE PREDICTION SYSTEM AND METHOD**

(30) Priority: 06.08.2021 KR 20210103572
(71) Applicant: Dong Woon Anatech Co., Ltd, Seocho-gu Seoul 06713 (KR)
(72) Inventor: JANG, In Su, Seoul 06716 (KR); KWON, Min Su, Seoul 06716 (KR); KYE, Ji Won, Seoul 06716 (KR); SHIM, Eun Hyun, Seoul 06716 (KR); KIM, Dong Cheol, Seoul 06716 (KR)
(74) Representative: AWA Sweden AB
(86) International application number: PCT/KR2022/011728
(87) International publication number: WO 2023/014197

(57) **Abstract**

It is disclosed a blood glucose prediction system and method. According to one example embodiment, the blood glucose prediction system may include a learning modeling unit for learning a blood glucose variability inference model to infer a correlation between a physical indicator and blood glucose variability, and learning a blood glucose inference model to infer a correlation between sugar of saliva and blood glucose; a target information acquiring unit for acquiring a physical indicator and sugar of saliva of a target; a blood glucose variability estimating unit for estimating blood glucose variability of the target by using the blood glucose variability inference model with the physical indicator of the target as an input parameter; and a blood glucose predicting unit for predicting blood glucose of the target by using the blood glucose inference model with the sugar of the saliva and the estimated blood glucose variability of the target as an input parameter.

## Description

This application claims the priority benefit of Korean Patent Application No. 10-2021-0103572, filed on August 06, 2021, in the Korean Intellectual Property Office, the disclosure of which is incorporated herein by reference.

### BACKGROUND

### 1. Field of the Invention

The below example embodiments relate to a blood glucose prediction system from sugar of saliva and a method thereof.

### 2. Description of Related Art

As an index for diagnosing diabetes, blood glucose which is a concentration of glucose in blood or urine glucose is generally used.

However, in a urine glucose test, since a person with impaired glucose tolerance, which is a borderline type between a diabetic patient and a healthy person, is classified as a diabetic patient, there is a problem in that accuracy of diabetes diagnosis is lowered.

Accordingly, blood glucose, which guarantees the accuracy of diabetes diagnosis, is mainly used. However, to measure such blood glucose, since there is a limit in which blood can be collected only by an invasive method, disadvantages such as inconvenience, pain, and the like of blood collection may be occurred.

Therefore, there is a need to propose a technology for overcoming the described limitation of the blood measurement and solving the disadvantages.

### SUMMARY

The example embodiments propose a blood glucose prediction system from sugar of saliva and a method thereof to overcome a limitation in which blood can be collected only by an invasive method and solve disadvantages such as inconvenience, pain, and the like of blood collection.

In more detail, the example embodiments propose a blood glucose prediction system from sugar of saliva by using a blood glucose inference model constructed by learning experiments for inferring a correlation between sugar of saliva and blood glucose based on artificial intelligence and a method thereof.

Particularly, the example embodiments propose a system for constructing a blood glucose inference model by considering blood glucose variability which is a factor affecting a correlation between sugar of saliva and blood glucose and a method thereof.

At this time, to solve disadvantages of inconvenience and complexity of blood glucose variability measurement, the example embodiments propose a system for estimating blood glucose variability from a physical indicator by using a blood glucose variability inference model constructed by learning experiments for inferring a correlation between a physical indicator and blood glucose variability of a target based on artificial intelligence and a method thereof.

However, technical problems to be solved by the present invention are not limited to above problems, and may be variously expanded within a scope that does not depart from technical ideas and areas of the present invention.

According to one example embodiment, a blood glucose prediction system may include a learning modeling unit for learning a blood glucose variability inference model to infer a correlation between a physical indicator and blood glucose variability, and learning a blood glucose inference model to infer a correlation between sugar of saliva and blood glucose; a target information acquiring unit for acquiring a physical indicator and sugar of saliva of a target; a blood glucose variability estimating unit for estimating blood glucose variability of the target by using the blood glucose variability inference model with the physical indicator of the target as an input parameter; and a blood glucose predicting unit for predicting blood glucose of the target by using the blood glucose inference model with the sugar of the saliva and the estimated blood glucose variability of the target as an input parameter.

According to one aspect of one example embodiment, the learning modeling unit may be configured to learn the blood glucose variability inference model to infer a correlation between the physical indicator and HOMA-IR and HOMA β-cell, and to infer a correlation between the HOMA-IR and HOMA β-cell and the blood glucose variability.

According to another aspect of one example embodiment, the learning modeling unit may be configured to learn the blood glucose variability inference model to infer a correlation between insulin resistance and insulin sensitivity calculated by the HOMA-IR and HOMA β-cell and the blood glucose variability.

According to another aspect of one example embodiment, the learning modeling unit may be configured to construct the blood glucose variability inference model by learning experiments for inferring the correlation between the physical indicator and the blood glucose variability based on artificial intelligence, and construct the blood glucose inference model by learning experiments for inferring the correlation between the sugar of the saliva and the blood glucose by considering the blood glucose variability based on artificial intelligence.

According to another aspect of one example embodiment, the physical indicator of the target may be configured to include BMI (Body Mass Index) and waist circumference of the target.

According to one example embodiment, a blood glucose prediction method may include acquiring a physical indicator and sugar of saliva of a target; estimating blood glucose variability of the target by using a blood glucose variability inference model with the physical indicator of the target as an input parameter - the blood glucose variability inference model is learned to infer a correlation of the physical indicator and the blood glucose variability-; and predicting blood glucose of the target by using a blood glucose inference model with the sugar of the saliva and the estimated blood glucose variability of the target as an input parameter - the blood glucose inference model is learned to infer a correlation between the sugar of the saliva and the blood glucose by considering the blood glucose variability-.

According to one aspect of one example embodiment, the blood glucose variability inference model may be configured to be learned to infer a correlation between the physical indicator and HOMA-IR and HOMA β-cell, and to infer a correlation between the HOMA-IR and HOMA β-cell and the blood glucose variability.

According to another aspect of one example embodiment, the blood glucose variability inference model may be configured to be learned to infer a correlation between insulin resistance and insulin sensitivity calculated by the HOMA-IR and HOMA β-cell and the blood glucose variability.

According to another aspect of one example embodiment, the blood glucose prediction method may further include constructing the blood glucose variability inference model by learning experiments for inferring the correlation between the physical indicator and the blood glucose variability based on artificial intelligence; and constructing the blood glucose inference model by learning experiments for inferring the correlation between the sugar of the saliva and the blood glucose by considering the blood glucose variability based on artificial intelligence.

According to one example embodiment, a blood glucose variability estimation system may include a learning modeling unit for learning a blood glucose variability inference model to infer a correlation of a physical indicator and blood glucose variability; a target information acquiring unit for acquiring a physical indicator of a target; and a blood glucose variability estimating unit for estimating blood glucose variability of the target by using the blood glucose variability inference model with the physical indicator of the target as an input parameter.

According to one aspect of one example embodiment, the learning modeling unit may be configured to learn the blood glucose variability inference model to infer a correlation between the physical indicator and HOMA-IR and HOMA β-cell, and to infer a correlation between the HOMA-IR and HOMA β-cell and the blood glucose variability.

According to another aspect of one example embodiment, the learning modeling unit may be configured to learn the blood glucose variability inference model to infer a correlation between insulin resistance and insulin sensitivity calculated by the HOMA-IR and HOMA β-cell and the blood glucose variability.

According to another aspect of one example embodiment, the learning modeling unit may be configured to construct the blood glucose variability inference model by learning experiments for inferring the correlation between the physical indicator and the blood glucose variability based on artificial intelligence.

According to another aspect of one example embodiment, the physical indicator of the target may be configured to include BMI (Body Mass Index) and waist circumference of the target.

According to one example embodiment, a blood glucose variability estimation method may include acquiring a physical indicator of a target; and estimating blood glucose variability of the target by using a blood glucose variability inference model with the physical indicator of the target as an input parameter - the blood glucose variability inference model is learned to infer a correlation of the physical indicator and the blood glucose variability-.

According to one aspect of one example embodiment, the blood glucose variability inference model may be configured to be learned to infer a correlation between the physical indicator and HOMA-IR and HOMA β-cell, and to infer a correlation between the HOMA-IR and HOMA β-cell and the blood glucose variability.

According to another aspect of one example embodiment, the blood glucose variability inference model may be configured to be learned to infer a correlation between insulin resistance and insulin sensitivity calculated by the HOMA-IR and HOMA β-cell and the blood glucose variability.

According to another aspect of one example embodiment, the blood glucose variability estimation method may further include constructing the blood glucose variability inference model by learning experiments for inferring the correlation between the physical indicator and the blood glucose variability based on artificial intelligence.

The example embodiments may overcome a limitation in which blood can be collected only by an invasive method in blood glucose measurement and solve disadvantages such as inconvenience, pain, and the like of blood collection by proposing a blood glucose prediction system from sugar of saliva and a method thereof.

In more detail, the example embodiments may propose a blood glucose prediction system from sugar of saliva by using a blood glucose inference model constructed by learning experiments for inferring a correlation between sugar of saliva and blood glucose based on artificial intelligence and a method thereof.

Particularly, the example embodiments may propose a system for constructing a blood glucose inference model by considering blood glucose variability which is a factor affecting a correlation between sugar of saliva and blood glucose and a method thereof.

At this time, the example embodiments may propose a system for estimating blood glucose variability from a physical indicator by using a blood glucose variability inference model constructed by learning experiments for inferring a correlation between a physical indicator and blood glucose variability of a target based on artificial intelligence and a method thereof, and through this, may solve disadvantages of inconvenience and complexity of blood glucose variability measurement.

However, effects of the present invention are not limited to above effects, and may be variously expanded within a scope that does not depart from technical ideas and areas of the present invention.

### BRIEF DESCRIPTION OF THE DRAWINGS

These and/or other aspects, features, and advantages of the disclosure will become apparent and more readily appreciated from the following description of embodiments, taken in conjunction with the accompanying drawings of which:
FIG. 1 is a drawing illustrating an example of a network environment according to one example embodiment;
FIG. 2 is a drawing for describing an internal configuration of an electronic device and a server according to one example embodiment;
FIG. 3 is a block diagram illustrating an example of a blood glucose prediction system implemented in a processor of a server according to one example embodiment;
FIG. 4 is a flow chart illustrating a blood glucose prediction method according to one example embodiment;
FIG. 5 is a concept diagram for describing a blood glucose variability inference model used in a blood glucose prediction method according to one example embodiment;
FIG. 6 is a block diagram illustrating an example of a blood glucose variability estimation system implemented in a processor of a server according to one example embodiment; and
FIG. 7 is a flow chart illustrating a blood glucose variability estimation method according to one example embodiment.

### DETAILED DESCRIPTION

Hereinafter, embodiments of the present invention will be described in detail with reference to the accompanying drawings. However, the present invention is not limited by the embodiments. In addition, same reference numerals on the drawings may refer to same components throughout.

In addition, terminologies used herein are defined to appropriately describe the embodiments of the present invention and thus may be changed depending on a user, the intent of an operator, or a custom in the field to which the present invention pertains. Accordingly, the terminologies must be defined based on the following overall description of this specification. In addition, the terms "comprising" and/or "comprises" used in the specification mean that the mentioned elements, steps, operations, and/or devices do not exclude existence or addition of one or more other elements, steps, operations, and/or devices. Also, although terms such as first, second, and the like are used in the specification to describe various areas, directions, shapes, and the like, these areas, directions, and shapes should not be limited by these terms. These terms are only used to distinguish one area, direction, or shape from another area, direction, or shape. Therefore, a part referred to as first part in one example embodiment may be referred to as second part in another example embodiment.

Moreover, it should be understood that various example embodiments of the present invention are not necessarily mutually exclusive although being different from each other. For example, specific shapes, structures, and characteristics described herein may be implemented in other embodiments without departing from technical idea and scope of the present invention in relation to one example embodiment. Besides, it should be understood that the location, arrangement, or configuration of individual components in each of presented categories of an example embodiment may be changed without departing from the technical idea and scope of the present invention.

Hereinafter, sugar of saliva means glucose included in saliva, and blood glucose means glucose included in blood. Also, hereinafter, acquiring sugar of saliva means acquiring information for sugar of saliva (e.g., concentration, index, numerical value, and the like of sugar of saliva), and predicting blood glucose means predicting information for blood glucose (e.g., concentration, index, numerical value, and the like of blood glucose).

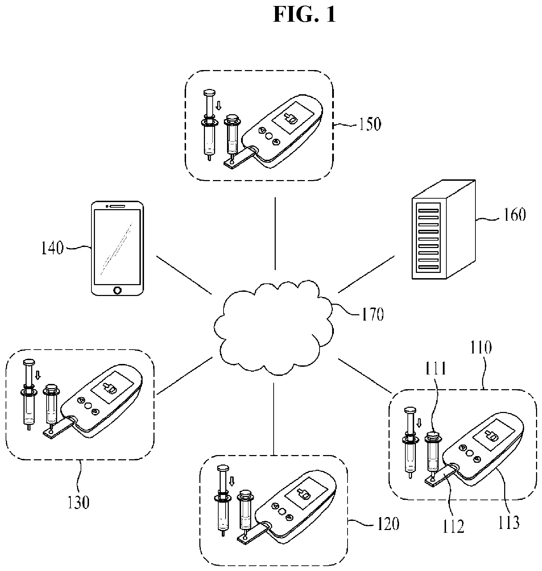

FIG. 1 is a drawing illustrating an example of a network environment according to one example embodiment. The network environment of FIG. 1 represents an example including a plurality of electronic devices 110, 120, 130, 140, 150, a server 160, and a network 170. Such FIG. 1 is one example for describing the invention, and the number of electronic devices and server is not limited as FIG. 1.

The plurality of electronic devices 110, 120, 130, 140, 150 are terminals possessed by each of specimen targets, and each of the plurality of electronic devices 110, 120, 130, 140, 150 may include a collecting device 111, a biosensor 112, and a measuring device 113 as a specimen measuring system.

The collecting device 111 may include a specimen collecting unit, a filter, and a compression tube as a device for collecting specimens (e.g., saliva). In case that the specimen is saliva, the collecting device 111 may remove an interfering substance through the filter in the compression tube from the collected saliva, and provide the saliva from which the interfering substance was removed to the outside, i.e., the biosensor 112.

The biosensor 112, which is an element performing a function of sensing information (e.g., glucose) to be measured from the specimen from which the interfering substance was removed in case that the specimen (e.g., saliva) collected by the collecting device 111 is inserted through the collecting device 111, may recognize the specimen based on a biosensor strip inserted to the measuring device 113 and a specimen recognizing signal received from the measuring device 113, and provide a responding signal for glucose included in the specimen through a specimen measuring signal applied separately from the specimen recognizing signal to the measuring device 113.

The measuring device 113 may determine whether the specimen is contacted to the biosensor 112 by providing the specimen recognizing signal to the biosensor 112 by recognizing inserting the biosensor 112, and then, may receive a responding signal of the specimen to be measured and measure the specimen by providing the specimen measuring signal if it is determined that the specimen is contacted.

Also, the measuring device 113 may include a communication module in order to communicate with the server 160 through the network 170. However, the plurality of electronic devices 110, 120, 130, 140, 150 including the measuring device 113 respectively may be configured to communicate with the server 160 by connecting to the network 170 through a terminal processed by the target (e.g., a smart phone, a mobile phone, a tablet PC, a navigation device, a computer, a laptop computer, a terminal for digital broadcasting, Personal Digital Assistants (PDF), a Portable Multimedia Player (PMP), and the like) instead of communicating with the server 160 by directly connecting to the network 170.

The communication method is not limited, and may include short-distance wireless communication between devices in addition to communication methods using a communication network (e.g., a mobile communication network, wired Internet, wireless Internet, and a broadcasting network) that the network 170 may include. For example, the network 170 may include one or more given networks of a personal area network (PAN), a local area network (LAN), a campus area network (CAN), a metropolitan area network (MAN), a wide area network (WAN), a broadband network (BBN), and the Internet. Furthermore, the network 170 may include one or more of network topologies, including a bus network, a star network, a ring network, a mesh network, a star-bus network, and a tree or hierarchical network, but is not limited thereto.

Hereinbefore, it was described that each of the plurality of electronic devices 110, 120, 130, 140, 150 includes the collecting device 111, the biosensor 112, and the measuring device 113, but it is not limited thereto, and it may be a terminal (e.g., a smart phone, a mobile phone, a tablet PC, a navigation device, a computer, a laptop computer, a terminal for digital broadcasting, Personal Digital Assistants (PDF), a Portable Multimedia Player (PMP), and the like) storing information relating to a physical indicator and sugar of saliva of a target .

The server 160 may be implemented with a computer device or a plurality of computer devices receiving information for the measured specimen (saliva) from the measuring device 113 of each of the plurality of electronic devices 110, 120, 130, 140, 150 by communicating with the plurality of electronic devices 110, 120, 130, 140, 150 through the network 170, and providing a blood glucose prediction service for predicting blood glucose of a target based on the received information for the specimen (saliva).

In other words, each of the plurality of electronic devices 110, 120, 130, 140, 150 may receive the blood glucose prediction service that the server 160 provides by connecting to the server 160 according to control of a pre-installed Operation System (OS) or at least one program (e.g., a browser or pre-installed application).

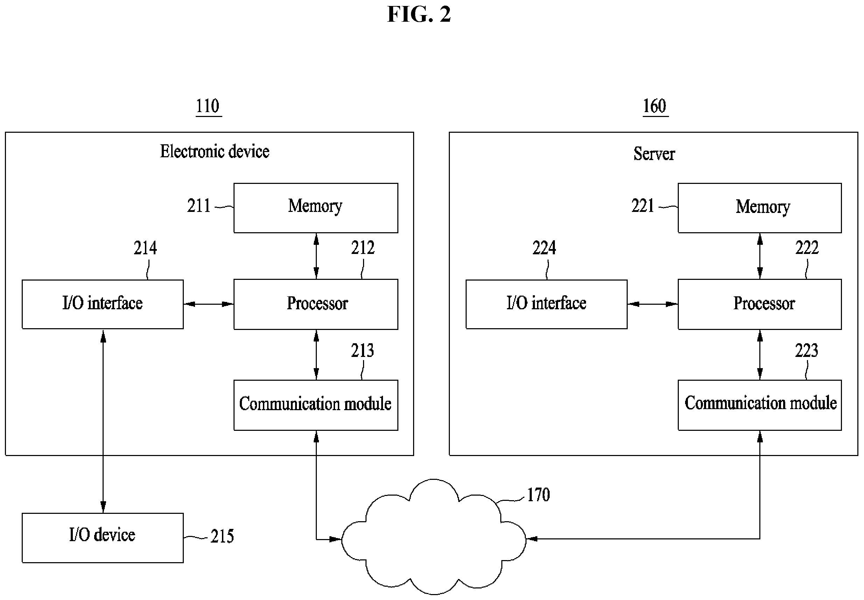

FIG. 2 is a drawing for describing an internal configuration of an electronic device and a server according to one example embodiment. In FIG. 2, it will be described a first electronic device 110 as an example of an electronic device possessed by a target receiving a blood glucose prediction service, and an internal configuration of the server 160 which is a main subject providing the blood glucose predication service by communicating with the first electronic device 110. Hereinafter, for the convenience of description for the first electronic device 110, the collecting device 111, the biosensor 112, and the measuring device 13 will be omitted.

The first electronic device 110 and the server 160 may include memories 211, 221, processors 212, 222, communication modules 213, 223, and input/output interfaces 214, 224. The memories 211, 221 may include a permanent mass storage device such as RAM (random access memory), ROM (read only memory), and disk drive as computer-readable recording medium. In addition, in the memories 211, 221, OS or at least one program code (e.g., a code for an application installed and driven in the first electronic device 110) may be stored. Such software components may be loaded from the computer-readable recording medium separate from the memories 211, 221. The separate computer-readable recording medium may include computer-readable recoding medium such as a floppy drive, a disc, a tape, a DVD/CD-ROM drive, a memory card, and the like. In other example embodiments, the software components may be loaded to the memories 211, 221 through the communication modules 213, 223, not through the computer-readable recording medium. For example, at least one computer program may be loaded to the memories 211, 221 based on a computer program (as an example, the above-described application) installed by files provided through the network 170 by developers or file distribution systems for distributing application installation files.

The processors 212, 222 may be configured to process computer program instructions by performing basic arithmetic, logic, and input/output operation. The instructions may be provided to the processors 212, 222 by the memories 211, 221 or the communication modules 213, 223. For example, the processors 212, 222 may be configured to execute the instructions received according to a program code stored in a recording device such as the memories 211, 221.

The communication modules 213, 223 may provide a function for communicating with the first electronic device 110 and the server 160 through the network 170, and may provide a function for communicating with another electronic device (e.g., the second electronic device 120, the third electronic device 130, the fourth electronic device 140, the fifth electronic device 150, and the like) or another server. As an example, a request (e.g., a request for a blood glucose prediction service) that the processor 212 of the first electronic device 110 generates according to a program code stored in a recording device such as the memory 211 may be transmitted to the server 160 through the network 170 according to control of the communication module 213. Conversely, a control signal or instruction, a content, a file, and the like which are provided according to control of the processor 222 of the server 160 may be received to the first electronic device 110 through the communication module 213 of the first electronic device 110 by going through the communication module 223 and the network 170. For example, the control signal or instruction and the like of the server 160 received through the communication module 213 may be transmitted to the processor 212 or the memory 211, and the content or the file and the like may be stored in the storage medium that the electronic device 110 may further include.

The input/output interface 214 may be a means for interfacing with an input/output device 215. For example, the input device may include a device such as a keyboard or a mouse, and the like, and an output device may include a device such as a display and the like for displaying communication session of an application. As another example, the input/output interface 214 may be a means for interfacing with a device in which an input function and an output function are integrated into a single function such as a touch screen. As more particular example, the processor 212 of the first electronic device 110 may display service screen or contents configured by using data provided by the server 160 or the second electronic device 120 on the display through the input/output interface 214 in processing the instruction of the computer program loaded to the memory 211. Likewise, the input/output interface 214 also may output the configured information by using data provided by the server 160 in processing the instruction of the computer program loaded to the memory 221 by the processor 222 of the server 160.

In addition, in other example embodiments, the first electronic device 110 and the server 160 may include much more components than the components of FIG. 2. However, there is no need to clearly illustrate most prior art components.

Hereinafter, it will be described a specific example embodiment of a blood glucose prediction method and system for providing a blood glucose service.

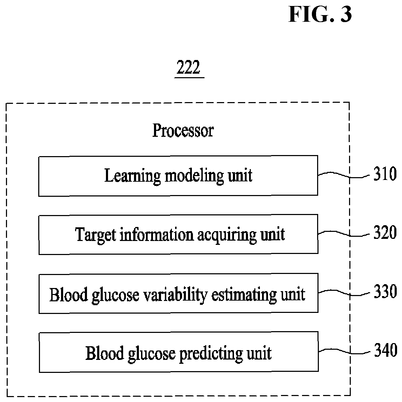

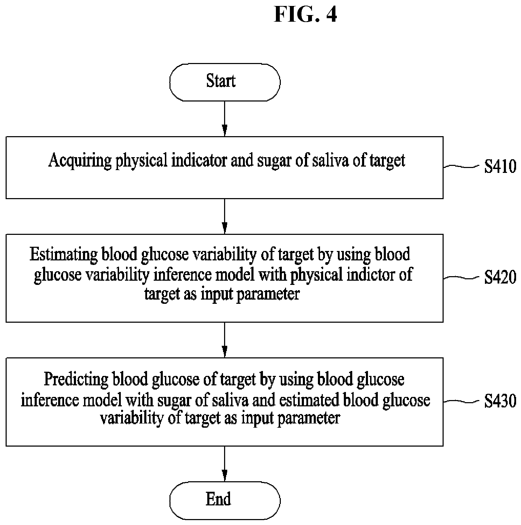

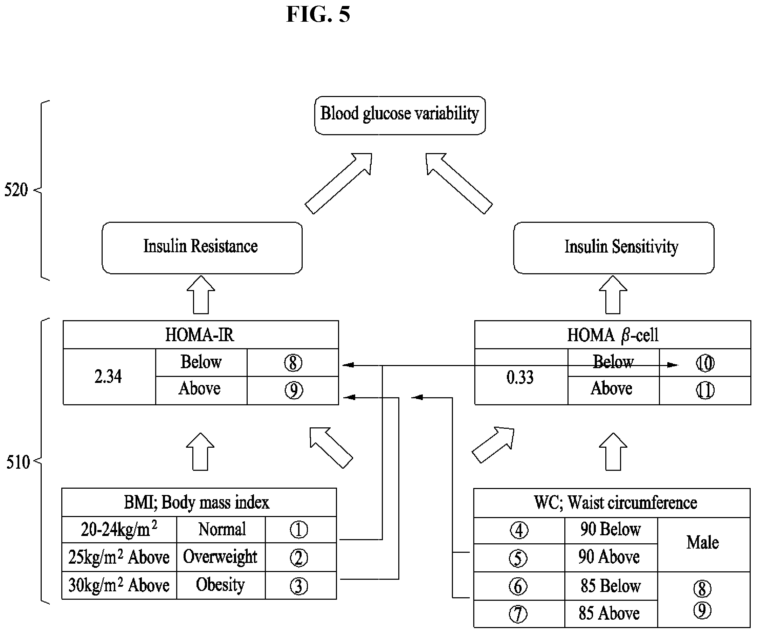

FIG. 3 is a block diagram illustrating an example of a blood glucose prediction system implemented in a processor of a server according to one example embodiment, FIG. 4 is a flow chart illustrating a blood glucose prediction method according to one example embodiment, and FIG. 5 is a concept diagram for describing a blood glucose variability inference model used in a blood glucose prediction method according to one example embodiment.

In the server 160 according to one example embodiment, a blood glucose prediction system implemented with a computer may be configured. The server 160, which is a main subject for providing a blood glucose prediction service for the plurality of electronic devices 110, 120, 130, 140, 150 which are clients, may provide the blood glucose prediction service to each of the plurality of electronic devices 110, 120, 130, 140, 150 by performing steps S410 to S430 shown in FIG. 4.

In order that the server 160 performs a blood glucose prediction method according to FIG. 4, the processor 222 of the server 160 may include a learning modeling unit 310, a target information acquiring unit 320, a blood glucose variability estimating unit 330, and a blood glucose predicting unit 340 as components as shown in FIG. 3. According to the example embodiment, the components of the processor 222 may be optionally included in or excluded from the processor 222. Also, according to the example embodiment, the components of the processor 222 may be separated or combined for expressions of functions of the processor 222. For example, at least part of the components of the processor 222 may be implemented in the processor 212 included in the first electronic device 110 which is a terminal of a first target, or implemented in a processor included in the second electronic device 120 which is a terminal of a second target.

Such processor 222 and the components of the processor 222 may control the server 160 to perform steps S410 to S430 included in the blood glucose prediction method of FIG. 4. For example, the processor 222 and the components of the processor 222 may be implemented to execute instructions according to a code of OS and a code of at least one program included in the memory 221.

Here, the components of the processor 222 may be expressions of different functions of the processor 222 performed by the processor 222 according to instructions provided by the program code stored in the server 160. For example, as a functional expression of the processor 222 for predicting blood glucose of a target, the blood glucose predicting unit 330 may be used.

Before step S410, the processor 222 may read required instructions from the memory 221 in which instructions related to control of the server 160 are loaded (not shown as a separate step in the drawing). In this case, the read instructions may include instructions for controlling the processor 222 to execute below described steps S410 to 430.

In addition, before step S410, the learning modeling unit 310 may learn and construct in advance a blood glucose variability inference model to infer a correlation between a physical indicator and blood glucose variability, and may learn and construct in advance a blood glucose inference model to infer a correlation between sugar of saliva and blood glucose by considering the blood glucose variability.

The correlation between the sugar of saliva and the blood glucose is affected by the blood glucose variability. Therefore, to accurately infer and define the correlation between the sugar of saliva and the blood glucose, the blood glucose variability should be considered.

Meanwhile, BMI (Body Mass Index) and waist circumference which are the physical indicator have a correlation with HOMA-IR and HOMA β-cell (hereinafter, the physical indicator typically includes BMI and waist circumference). Also, insulin resistance and insulin sensitivity may be calculated from the HOMA-IR and HOMA β-cell, and the insulin resistance and the insulin sensitivity have a correlation with the blood glucose variability.

By using such characteristics, while the learning modeling unit 310 may construct the blood glucose inference model by learning experiments for inferring the correlation between the sugar of saliva and the blood glucose by considering the blood glucose variability, it may also construct the blood glucose variability inference model for inferring the blood glucose variability to be reflected to the blood glucose inference model. For example, the learning modeling unit 310 may learn and construct the blood glucose variability inference model for inferring a correlation between the physical indicator and the HOMA-IR and HOMA β-cell 510, and a correlation between insulin resistance and insulin sensitivity calculated by the HOMA-IR and HOMA β-cell and the blood glucose variability 520 through experiments as shown in FIG. 5.

In other words, the learning modeling unit 310 may construct the blood glucose variability inference model by learning the experiments for inferring the correlation between the physical indicator and the blood glucose variability based on artificial intelligence, and construct the blood glucose inference model by learning the experiments for inferring the correlation between the sugar of saliva and the blood glucose by considering the blood glucose variability based on artificial intelligence.

In learning process of each of such blood glucose variability inference model and blood glucose inference model, various known machine learning algorithms may be used.

In step S410, the target information acquiring unit 320 may acquire the physical indicator and the sugar of saliva of the target. For example, the target information acquiring unit 320 may receive and acquire information for the saliva measured through the measuring device 113 of each of the plurality of electronic devices 110, 120, 130, 140, 150 as above described, and may receive input and acquire the physical indicator of each of targets from each of the plurality of electronic devices 110, 120, 130, 140, 150.

In step S420, the blood glucose variability estimating unit 330 may estimate the blood glucose variability of the target by using the blood glucose variability inference model with the physical indicator of the target as an input parameter.

In step S430, the blood glucose predicting unit 340 may predict the blood glucose of the target by using the blood glucose inference model with the sugar of saliva and the estimated blood glucose variability of the target as an input parameter. The predicted blood glucose of the target may be provided to an electronic device corresponding to the target.

Likewise, the blood glucose prediction system and method according to one example embodiment may overcome a limitation in which blood can be collected only by an invasive method by predicting the blood glucose from the sugar of saliva by using the blood glucose inference model and solve disadvantages such as inconvenience, pain, and the like of blood collection, and improve accuracy of blood glucose prediction by considering the blood glucose variability in the blood glucose inference model.

In addition, the blood glucose prediction system and method according to one example embodiment may solve disadvantages of inconvenience and complexity of the blood glucose variability measurement by estimating the blood glucose variability to be used as an input parameter in the blood glucose inference model through the blood glucose variability inference model.

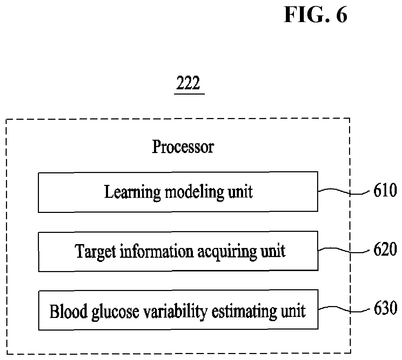

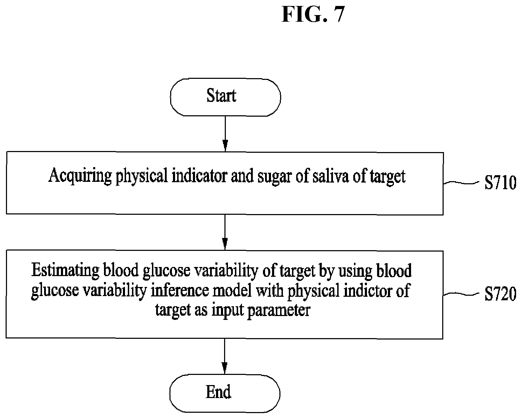

Hereinbefore, the blood glucose prediction system and the method thereof are described, but the present invention may independently apply and use only the blood glucose variability estimation. The detailed description thereof will be described below with reference to FIGS. 6 and 7.

Furthermore, hereinbefore, estimating the blood glucose from the sugar of saliva was described, but estimating the sugar of saliva from the blood glucose also may be possible through the blood glucose prediction system according to one example embodiment. In this case, the target information acquiring unit 320 may respond to acquiring the blood glucose instead of the sugar of saliva, and the blood glucose predicting unit 340 may predict the sugar of saliva from the acquired blood glucose.

FIG. 6 is a block diagram illustrating an example of a blood glucose variability estimation system implemented in a processor of a server according to one example embodiment, and FIG. 7 is a flow chart illustrating a blood glucose variability estimation method according to one example embodiment.

Referring to FIGS. 6 and 7, in the server 160 according to one example embodiment, a blood glucose variability estimation system which is implemented by a computer may be configured. The server 160, which is a main subject for providing a blood glucose variability estimation service to the plurality of electronic devices 110, 120, 130, 140, 150 that are clients, may provide the blood glucose estimation service to each of the plurality of electronic devices 110, 120, 130, 140, 150 by performing steps S710 and S720 shown in FIG. 7.

In order that the server 160 may perform the blood glucose estimation method according to FIG. 7, the processor 222 of the server 160 may include a learning modeling unit 610, a target information acquiring unit 620, and a blood glucose variability estimating unit 630, as shown in FIG. 6. According to example embodiments, the components of the processor 222 may be optionally included in or excluded from the processor 222. Also, the components of the processor 222 may be separated or combined for expressions of functions of the processor 222. For example, at least part of the components of the processor 222 may be implemented in the processor 212 included in the first electronic device 110 which is a terminal of a first target, or implemented in a processor included in the second electronic device 120 which is a terminal of a second target.

Such processor 222 and components of the processor 222 may control the server 160 to perform steps S710 and S720 included in the blood glucose variability estimation method of FIG. 7. For example, the processor 222 and the components of the processor 222 may be implemented to execute instructions according to a code of OS and a code of at least one program included in the memory 221.

Here, the components of the processor 222 may be expressions of different functions of the processor 222 performed by the processor 222 according to instructions provided by the program code stored in the server 160. For example, as a functional expression of the processor 222 for estimating blood glucose variability of a target, the blood glucose variability estimating unit 630 may be used.

Before step S710, the processor 222 may read required instructions from the memory 221 in which instructions related to control of the server 160 are loaded (not shown as a separate step in the drawing). In this case, the read instructions may include instructions for controlling the processor 222 to execute below described steps 710 and 720.

In addition, before step S710, the learning modeling unit 610 may learn and construct in advance a blood glucose variability inference model to infer a correlation between a physical indicator and blood glucose variability.

BMI (Body Mass Index) and waist circumference which are the physical indicator have a correlation with HOMA-IR and HOMA β-cell (hereinafter, the physical indicator typically includes BMI and waist circumference). Also, insulin resistance and insulin sensitivity may be calculated from the HOMA-IR and HOMA β-cell, and the insulin resistance and the insulin sensitivity have a correlation with the blood glucose variability.

By using such characteristics, while the learning modeling unit 610 may construct the blood glucose variability inference model for inferring the blood glucose variability. For example, the learning modeling unit 610 may learn and construct the blood glucose variability inference model for inferring the correlation between the physical indicator and the HOMA-IR and HOMA β-cell 510, and inferring the correlation between insulin resistance and insulin sensitivity calculated by the HOMA-IR and HOMA β-cell and the blood glucose variability 520 through experiments as shown in FIG. 5.

In other words, the learning modeling unit 610 may construct the blood glucose variability inference model by learning the experiments for inferring the correlation between the physical indicator and the blood glucose variability based on artificial intelligence.

In learning process of the blood glucose variability inference model, various known machine learning algorithms may be used.

In step S710, the target information acquiring unit 620 may acquire the physical indicator of the target. For example, the target information acquiring unit 620 may receive input and acquire the physical indicator of each of targets from each of the plurality of electronic devices 110, 120, 130, 140, 150.

In step S720, the blood glucose variability estimating unit 630 may estimate the blood glucose variability of the target by using the blood glucose variability inference model with the physical indicator of the target as an input parameter.

Likewise, the blood glucose estimation system and method according to one example embodiment may solve disadvantages of inconvenience and complexity of the blood glucose variability measurement by estimating the blood glucose variability through the blood glucose variability inference model.

As described above, the estimated blood glucose variability may be used not only in the above described blood glucose estimation system and method but also in various works and fields.

The device described herein may be implemented using hardware components, software components, and/or a combination thereof. For example, the device and components described in the example embodiments may be implemented using one or more general-purpose or special purpose computers, such as, for example, a processor, a controller and an ALU (arithmetic logic unit), a digital signal processor, a microcomputer, a FPA (field programmable array), a PLU (programmable logic unit), a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will be appreciated that a processing device may include multiple processing elements and/or multiple types of processing elements. For example, a processing device may include multiple processors or a processor and a controller. In addition, different processing configurations are possible, such as parallel processors.

The software may include a computer program, a piece of code, an instruction, or some combination thereof, for independently or collectively instructing or configuring the processing device to operate as desired. Software and/or data may be embodied in any type of machine, component, physical or virtual equipment, computer storage medium or device to provide instructions or data to or being interpreted by the processing device. The software also may be distributed over network coupled computer systems so that the software is stored and executed in a distributed fashion. In particular, the software and data may be stored by one or more computer readable recording mediums.

The method according to the embodiment may be implemented in the form of a program instruction that maybe performed through various computer means, and may be recorded in a computer readable medium. The computer readable medium may include a program instruction, a data file, and a data structure alone or in combination thereof. The program instruction recorded in the medium may be designed or configured particularly for the embodiment or may be a usable one known to those skilled in computer software. An example of the computer readable recording medium may include magnetic media such as a hard disk, a floppy disk, and a magnetic tape, optical recording media such as a CD-ROM and a DVD, magneto-optical media such as a floptical disk, and hardware devices that are particularly configured to store and perform a program instruction, such as a ROM, a RAM, and a flash memory. Further, an example of the program instruction may include high-level language codes which may be executed by a computer using an interpreter as well as machine languages created by using a compiler.

As described above, although the embodiments have been described in connection with the limited embodiments and the drawings, those skilled in the art may modify and change the embodiments in various ways from the description. For example, proper results may be achieved although the aforementioned descriptions are performed in order different from that of the described method and/or the aforementioned elements, such as the system, configuration, device, and circuit, are coupled or combined in a form different from that of the described method or replaced or substituted with other elements or equivalents.

Accordingly, other implementations, other embodiments, and the equivalents of the claims fall within the scope of the claims.

## Claims

1. A blood glucose prediction system, comprising:
a learning modeling unit for learning a blood glucose variability inference model to infer a correlation between a physical indicator and blood glucose variability, and learning a blood glucose inference model to infer a correlation between sugar of saliva and blood glucose;
a target information acquiring unit for acquiring a physical indicator and sugar of saliva of a target;
a blood glucose variability estimating unit for estimating blood glucose variability of the target by using the blood glucose variability inference model with the physical indicator of the target as an input parameter; and
a blood glucose predicting unit for predicting blood glucose of the target by using the blood glucose inference model with the sugar of the saliva and the estimated blood glucose variability of the target as an input parameter.

2. The blood glucose prediction system of claim 1, wherein the learning modeling unit is configured to learn the blood glucose variability inference model to infer a correlation between the physical indicator and HOMA-IR and HOMA β-cell, and to infer a correlation between the HOMA-IR and HOMA β-cell and the blood glucose variability.

3. The blood glucose prediction system of claim 2, wherein the learning modeling unit is configured to learn the blood glucose variability inference model to infer a correlation between insulin resistance and insulin sensitivity calculated by the HOMA-IR and HOMA β-cell and the blood glucose variability.

4. The blood glucose prediction system of claim 1, wherein the learning modeling unit is configured to construct the blood glucose variability inference model by learning experiments for inferring the correlation between the physical indicator and the blood glucose variability based on artificial intelligence, and construct the blood glucose inference model by learning experiments for inferring the correlation between the sugar of the saliva and the blood glucose by considering the blood glucose variability based on artificial intelligence.

5. The blood glucose prediction system of claim 1, wherein the physical indicator of the target is configured to include BMI (Body Mass Index) and waist circumference of the target.

6. A blood glucose prediction method, comprising:
acquiring a physical indicator and sugar of saliva of a target;
estimating blood glucose variability of the target by using a blood glucose variability inference model with the physical indicator of the target as an input parameter - the blood glucose variability inference model is learned to infer a correlation of the physical indicator and the blood glucose variability-, and
predicting blood glucose of the target by using a blood glucose inference model with the sugar of the saliva and the estimated blood glucose variability of the target as an input parameter - the blood glucose inference model is learned to infer a correlation between the sugar of the saliva and the blood glucose by considering the blood glucose variability-.

7. The blood glucose prediction method of claim 6, wherein the blood glucose variability inference model is configured to be learned to infer a correlation between the physical indicator and HOMA-IR and HOMA β-cell, and to infer a correlation between the HOMA-IR and HOMA β-cell and the blood glucose variability.

8. The blood glucose prediction method of claim 7, wherein the blood glucose variability inference model is configured to be learned to infer a correlation between insulin resistance and insulin sensitivity calculated by the HOMA-IR and HOMA β-cell and the blood glucose variability.

9. The blood glucose prediction method of claim 6, further comprising:
constructing the blood glucose variability inference model by learning experiments for inferring the correlation between the physical indicator and the blood glucose variability based on artificial intelligence, and
constructing the blood glucose inference model by learning experiments for inferring the correlation between the sugar of the saliva and the blood glucose by considering the blood glucose variability based on artificial intelligence.

10. A blood glucose variability estimation system, comprising:
a learning modeling unit for learning a blood glucose variability inference model to infer a correlation of a physical indicator and blood glucose variability;
a target information acquiring unit for acquiring a physical indicator of a target; and
a blood glucose variability estimating unit for estimating blood glucose variability of the target by using the blood glucose variability inference model with the physical indicator of the target as an input parameter.

11. The blood glucose variability estimation system of claim 10, wherein the learning modeling unit is configured to learn the blood glucose variability inference model to infer a correlation between the physical indicator and HOMA-IR and HOMA β-cell, and to infer a correlation between the HOMA-IR and HOMA β-cell and the blood glucose variability.

12. The blood glucose variability estimation system of claim 10, wherein the learning modeling unit is configured to learn the blood glucose variability inference model to infer a correlation between insulin resistance and insulin sensitivity calculated by the HOMA-IR and HOMA β-cell and the blood glucose variability.

13. The blood glucose variability estimation system of claim 10, wherein the learning modeling unit is configured to construct the blood glucose variability inference model by learning experiments for inferring the correlation between the physical indicator and the blood glucose variability based on artificial intelligence.

14. The blood glucose variability estimation system of claim 10, wherein the physical indicator of the target is configured to include BMI (Body Mass Index) and waist circumference of the target.

15. A blood glucose variability estimation method, comprising:
acquiring a physical indicator of a target; and
estimating blood glucose variability of the target by using a blood glucose variability inference model with the physical indicator of the target as an input parameter - the blood glucose variability inference model is learned to infer a correlation of the physical indicator and the blood glucose variability-.

16. The blood glucose variability estimation method of claim 15, wherein the blood glucose variability inference model is configured to be learned to infer a correlation between the physical indicator and HOMA-IR and HOMA β-cell, and to infer a correlation between the HOMA-IR and HOMA β-cell and the blood glucose variability.

17. The blood glucose variability estimation method of claim 16, wherein the blood glucose variability inference model is configured to be learned to infer a correlation between insulin resistance and insulin sensitivity calculated by the HOMA-IR and HOMA β-cell and the blood glucose variability.

18. The blood glucose variability estimation method of claim 15, further comprising:
constructing the blood glucose variability inference model by learning experiments for inferring the correlation between the physical indicator and the blood glucose variability based on artificial intelligence.
